# EUROPEAN PATENT APPLICATION

(11) **EP 1 757 267 A2**
(43) Date of publication of application: **28.02.2007**
(21) Application number: 06017751.6
(22) Date of filing: 25.08.2006
(51) Int. Cl.: A61K 9/00, A61K 9/20

(54) **Mucosal delivery tablet**

(30) Priority: 26.08.2005 US 212437
(71) Applicant: National Starch and Chemical Investment Holding Corporation, New Castle, Delaware 19720 (US)
(72) Inventor: Foreman, Paul B., Somerville, NJ 08807 (US); Zhang, Yeli, Hillsborough, NJ 08844 (US); Puri, Reema, Bridgewater, NJ 08807 (US)
(74) Representative: Held, Stephan

(57) **Abstract**

Mucoadhesive tablets have a convex surface, a diameter to cup depth ratio of 4-20 and a cup depth to edge thickness ratio of greater than 0.75 swiftly adheres and conforms to the contacting tissue. The tablets are used to administer actives such as, for example, benzocaine.

## Description

### Field of the Invention

The invention relates to a bioadhesive tablet shaped to provide improved adhesion to the mucosa, especially to the oral mucosa.

### Background of the Invention

Bioadhesive formulations are designed for mucosal adhesion. The most commonly used bioadhesive forms are pastes, ointments, lozenges, tablets, patches and gels. Such formulations are used to enhance a local or systemic action of drugs in the treatment of systemic diseases or diseases of the oropharynx or other parts of the human body.

Bioadhesive tablets for local oral or systemic drug delivery are preferably placed, on the gingiva for example, so as to minimize stress from movement of the mouth or from contact with the tongue and teeth. However, there are some conditions, a canker or cold sore for example, where the medication must be applied directly to the sore wherever it happens to be within the mouth. In this case, a conventional flat or cupped tablet is liable to become unintentionally dislodged by movement of the mouth. There is thus a need in the art for tablet forms that mitigate this problem. The current invention addresses this need.

### Summary of the Invention

The invention provides a cupped or convex-shaped tablet with a thin edge. Use of such a tablet design reduces contact with the tongue or teeth and provides for rapid hydration around the perimeter to swiftly adhere and conform to the contacting tissue. At the same time, the cupped shape resists brittle fracture during manufacture, packaging, dispensing and handling.

One embodiment of the invention provides a specially shaped tablet. The specially shaped tablet has a convex (cupped) surface on one or both sides and a substantially reduced edge thickness compared with a conventional tablet design. The tablet of the invention has a ratio of tablet diameter D to the cup depth *d* (*D*/*d*) of 4-20 and a ratio of cup depth d to edge thickness *h* (*d*/*h*) of greater than 0.75. The tablet of the invention is especially well suited for use as a bioadhesive tablet for the systemic and/or local administration of an active agent. In one aspect of this embodiment, the specially shaped tablet comprises a pharmaceutical, nutraceutical or cosmeceutical agent. In one aspect the tablet contains benzocaine. In another aspect the tablet contains an oral hygiene agent. The tablet of the invention will comprises an active ingredient present in an amount sufficient to impart a desired action or dose upon administration of a single dosage form of the tablet.

Tablets of the invention preferred for mucoadhesion and administration of the active to the mucosa will comprise a bioadhesive carrier and an active to be delivered. Preferred bioadhesive carriers comprise a polysaccharide and a polycarboxylated polymer. More preferably, the bioadhesive carrier comprises about 5 % by weight to about 95 % by weight of at least one synthetic polycarboxylated polymer and from about 5 % by weight to about 95 % by weight of at least one polysaccharide. Even more preferably, the bioadhesive carrier will comprise from about 75 % by weight to about 95 % by weight of a starch and from about 5 % by weight to about 25 % by weight of cross-linked poly(acrylic acid). In a particularly preferred embodiment of the invention, these polymer solutions are co-spray dried to form an intimate mixture of these components.

Another embodiment of the invention is directed to a method of preparing a drug delivery system comprising mixing together a bioadhesive carrier composition and the active ingredient and applying pressure to form a tablet having a ratio of tablet diameter *D* to the cup depth *d* (*D*/*d*) of 4-20 and a ratio of cup depth *d* to edge thickness *h* (*d*/*h*) of greater than 0.75. Preferably, the bioadhesive composition used to prepare the drug delivery system is in the form of a powder.

Still another embodiment of the invention provides a method of delivering a drug or medication to an individual in need of or desiring such drug or medication via this specially shaped tablet. The invention provides a method of administering an active ingredient to an individual needing or desiring the active. The method comprises applying an active-containing tablet to the mucosa of an individual, where after the active is released. The active ingredient is present in the tablet in an amount sufficient to impart a desired action or to deliver a predetermined dose upon administration of a single dosage form of the active-containing tablet.

Yet another embodiment of the invention provides a method of treating a sore within the mouth by placing a specially shaped bioadhesive tablet comprising an active agent in contact with the sore. The active agent may, for example, be a local anaesthetic such as benzocaine, an antifungal such as nystatin, or an antiviral such as acyclovir.

### Brief Description of the Drawing Figures

Figure 1 shows the profile of a two-sided convex tablet in accordance with the invention.
Figure 2 shows the profile of a one-sided convex tablet in accordance with the invention.
Figure 3 is a drawing of the profiles of conventional convex and flat-faced bevel-edged tablets.

### Detailed Description of the Invention

While conventional flat bioadhesive tablets may provide sufficient adhesion when placed, for example on the gingiva, when a condition dictates placement elsewhere in the mouth a conventional flat or cupped tablet is liable to become unintentionally dislodged by movement of the mouth.

It has now been discovered that use of a cupped tablet with a thin edge reduces contact with the tongue or teeth and provides for rapid hydration around the perimeter to swiftly adhere and conform the tablet to the contacting tissue. At the same time, the cupped shape resists brittle fracture during manufacture, packaging, dispensing and handling.

In the practice of the invention, a concave punch is used, thereby forming a tablet with a convex surface, and the tablet edge thickness is reduced to a minimum and is much thinner than conventional tablets heretofore known and used in the art.

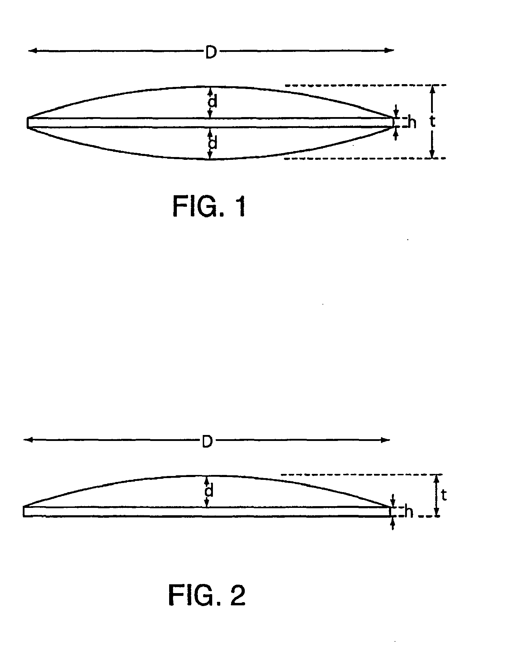

It has been found that the thicker center reduces brittleness and the thin edge hydrates more rapidly, adhering and conforming to the mouth and resisting dislodging by the tongue or teeth. The tablet has a convex profile. By convex profile means that when viewed edge-to-edge, at least one side is convex in shape. The tablet can be convex on both sides, as shown in drawing Figure 1, or convex on only one side, as shown in drawing Figure 2.

Preferred tablets of the invention are made of a bioadhesive composition in combination with certain ingredients that provides a therapeutic and/or cosmetic effect. Such ingredients may be therapeutic agents and/or cosmeceutical agents (e.g., breath freshening agents) for use in the oral cavity.

A bioadhesive composition refers to the component or components that provides bioadhesive properties to a bioadhesive system.

Bioadhesive properties mean that adhesive properties are developed on contact with animal or human mucosa, skin or body tissue, including traumatized tissue (e.g., severe burn), or vegetable or plant tissues wherein some water or an aqueous solution is present. Non-limiting examples of types of bioadhesives include intestinal, nasal, buccal, sub-lingual, vaginal, rectal and ocular bioadhesives.

Bioadhesion, as used herein, is intended to mean the ability of a material (synthetic or biological) to adhere to biological tissue. Bioadhesion stages can be summarized as follows. First an intimate contact must exist between the bioadhesive and the receptor tissue. Such contact results either from a good wetting of the bioadhesion surface or from the swelling of the bioadhesive. When contact is established, the penetration of the bioadhesive into the crevice of the tissue surface then takes place, or there is interpenetration of bioadhesive chains with those of the mucus, and there is formation of weak chemical bonds between entangled chains. A general description of bioadhesion may be found in Bioadhesive Drug Delivery Systems, 1999, pp. 1-10, Published by Marcel Dekker.

The bioadhesive compositions of the invention are particularly useful as sustained or controlled release preparations comprising the bioadhesive composition and an active ingredient. The bioadhesive compositions may be used for both systemic and local administration of active ingredient.

The controlled release preparations of the invention find use in the administration of therapeutic agents to individuals in need of, or desirous of, the therapeutic agent. The term "individual" is used herein in its broadest sense and includes animals (both human and non-human, including companion animals such as dogs, cats and horses and livestock such as cattle and swine) and plants (both agricultural and horticultural applications).

Controlled release, as used herein, is intended to mean a method and composition for making an active ingredient available to the biological system of a host. Controlled release includes the use of instantaneous release, delayed release, and sustained release. "Instantaneous release" refers to immediate release to the biosystem of the host. "Delayed release" means the active ingredient is not made available to the host until some time delay after administration. "Sustained Release" generally refers to release of active ingredient whereby the level of active ingredient available to the host is maintained at some level over a period of time. The method of effecting each type of release can be varied. For example, the active ingredient can be associated physically and/or chemically with a surfactant, a chelating agent, etc. Alternatively, the active ingredient can be masked by a coating, a laminate, etc. Regardless of the method of providing the desired release pattern, the present invention contemplates delivery of a controlled release system that utilizes one or more of the "release" methods and compositions. Moreover, the present invention can be an element of the release method and/or composition, especially with respect to sustained release systems.

The bioadhesive composition of the present invention may take up and controllably release active components such as drugs. Active components may be added using any of the known methods described in the prior art, and such addition may be carried out during and/or after the production of the bioadhesive composition. Typical active components may include, but are not limited to, a therapeutic substance or a pharmaceutically active agent such as a drug, a non-therapeutic substance such as a cosmetic, a breath freshener and the like, a local or general anesthetic or pain killer, or an opiate, a vaccine, an antigen, a microorganism, a sterilizing substance, a contraceptive composition, a protein or peptide such as insulin or calcitonin, an insecticide, a herbicide, a hormone such as a growth hormone or a seed germination hormone, a steroid, a toxin, or a marker substance. Classes of actives that can be administered using the tablet of the invention include α-adrenoreceptor agonists, β-adrenoreceptor agonists, α-adrenoreceptor blockers, β-adrenoreceptor blockers, anabolics, analgesics (narcotics and non-narcotics), androgens, anesthetics, antiallergics, antiandrogens, antianginals, antiarrhythmics, antidiabetics, antihistamics, anti-migraine agents, bronchodialators, gestagens and vasodilators. Included are actives for sedation, insomnia, dental and gum disease, motion sickness, emetic, nicotine addiction, bladder control and central nervous system diseases.

A non-limiting list of possible active components includes hydrochlorothiazide, acetazolamide, acetylsalicyclic acid, allopurinol, alprenolol, amiloride, antiarrhythmics, antibiotics, antidiabetics, antiepileptics, anticoagulants, antimycotics, atenolol, bendroflumethiazide, benzbromarone, benzocaine, benzthiazide, betamethasone, bronchodilators, buphenine, bupranolol, calcitonin, chemotherapeutics, chlordiazepoxide, chlorquine, chloro thiazide, chlorpromazine, chlortalidone, clenbuterol, clomipramine, clonidine, co-dergocrine, cortisone, dexamethasone, dextropropoxyphene, diazepam, diazoxide, diclofenac, diclofenamide, digitalisglycoside, dihydralazine, dihydroergotamine, diltiazem, iron salt, ergotamine, ethacrynic acid, ethinylestradiol, ethoxzolamide, fenoterol, fludrocortisone, fluphenazine, fluorosemide, gallopamil, guanethidine, hormones, hydrochlorothiazide, hydrocortisone, hydroflumethiazide, insulin, immunosuppressives, ibuprofen, imipramine, indomethacine, coronartherapeutics, levodopa, lithium salt, magnesium salt, medroxyprogesteron acetate, manadione, methaqualone, 8-methoxypsoralen, methylclothiazide, methyldopa, methylprednisolone, methyltestosterone, methylthiouracil, methylxanthine, metipranolol, miconazole nitrate, molsidomine, morphine, naproxen, nicotine, nicergline, nifedipine, norfenefrine, oxyphenbutazone, papaverine, parmathasone, pentobarbital, perphenazine, phenobarbital, phenylbutazone, phytomenadione, pirenzepine, polythiazide, prazosine, prednisolone, prednisone, probenecid, propranolol, propylthiouracil, rescinnamine, reserpine, secbutabarbital, secobarbital, spironolactone, sulfasalazine, sulfonamide, testosterone, theophylline, thioridazine, triamcinolon, triamteren, trichloromethiazide, trifluoperazine, trifluopromazine, tuberculostatic, verapamil, virustatics, zytostatics, bromocriptine, bromopride, carbidopa, carbocromen, quinine, chlorprothixene, cimetidine, clofibrat, cyclizine, desipramine, disulfiram, domperidone, doxepine, fenbufen, flufenamine acid, flunarizine, gemfibrocil, haloperidol, ketoprofen, labetalol, lorazepam, mefenamine acid, melperone, metoclopramide, nortriptyline, noscapine, oxprenolol, oxymetholone, pentazocine, pethidine, stanozolol, sulindac, sulpiride, tiotixen.

Common nutraceutical actives which may be administered using the tablets of the invention include, but are not limited to, functional foods, dietary supplements, herbal products; including antioxidants, immune enhancers, cardiovascular health enhancers, healthy joints and cartilage enhancers, memory and mental enhancers, women's health enhancers, mood and emotional enhancers, and weight loss enhancers. These include, but not are not limited to caffeine, folic acid, beta-carotene, lycopene, valerian, ginseng, vitamin E, herbal teas (e.g., green tea), and natural biological flora.

Flavorings can also be administered using the tablets of the invention. These flavorings may be chosen from synthetic flavor oils and flavoring aromatics, and/or oils, oleo resins and extracts derived from plants, leaves, flowers, fruits and so forth, and combinations thereof. Representative flavor oils include: spearmint oil, cinnamon oil, peppermint oil, clove oil, bay oil, thyme oil, cedar leaf oil, oil of nutmeg, oil of sage, and oil of bitter almonds. Also useful are artificial, natural or synthetic fruit flavors such as vanilla, chocolate, coffee, cocoa and citrus oil, including lemon, orange, grape, lime and grapefruit and fruit essences including apple, pear, peach, strawberry, raspberry, cherry, plum, pineapple, apricot and so forth. These flavorings can be used individually or in admixture. Commonly used flavors include mints such as peppermint, artificial vanilla, cinnamon derivatives, and various fruit flavors, whether employed individually or in admixture. Flavorings such as aldehydes and esters including cinnamyl acetate, cinnamaldehyde, citral, diethylacetal, dihydrocarvyl acetate, eugenyl formate, p-methylanisole, and so forth may also be used. Generally, any flavoring or food additive, such as those described in Chemicals Used in Food Processing, publication 1274 by the National Academy of Sciences, pages 63-258, may be used. Further examples of aldehyde flavorings include, but are not limited to acetaldehyde (apple); benzaldehyde (cherry, almond); cinnamic aldehyde (cinnamon); citral, i.e., alpha citral (lemon, lime); neral, i.e. beta citral (lemon, lime); decanal (orange, lemon); ethyl vanillin (vanilla, cream); heliotropine, i.e., piperonal (vanilla, cream); vanillin (vanilla, cream); alpha-amyl cinnarnaldehyde (spicy fruity flavors); butyraldehyde (butter, cheese); valeraldehyde (butter, cheese); citronellal (modifies, many types); decanal (citrus fruits); aldehyde C-8 (citrus fruits); aldehyde C-9 (citrus fruits); aldehyde C-12 (citrus fruits); 2-ethyl butyraldehyde (berry fruits); hexenal, i.e. trans-2 (berry fruits); tolyl aldehyde (cherry, almond); veratraldehyde (vanilla); 2,6-dimethyl-5-heptenal, i.e. melonal (melon); 26-dimethyloctanal (green fruit); and 2-dodecenal (citrus, mandarin); cherry, grape; mixtures thereof, and the like.

The amount of flavoring employed is normally a matter of preference subject to such factors as flavor type, individual flavor, strength desired and taste masking required. Thus, the amount may be varied in order to obtain the result desired in the final product. Such variations are within the capabilities of those skilled in the art without the need for undue experimentation.

The active is added in such amounts such that the final active-containing single dosage tablet comprises a pre-determined effective amount. By effective amount is meant that the active agent is present in amounts required to impart a desired action or therapeutic dose, such as a desired organoleptic, physiological, therapeutic, nutraceutical, or flavor effect. The active is present in an amount sufficient, also referred to herein as an effective amount, to bring about a desired result, e.g., a desired therapeutic result in the treatment of a condition. An effective amount of a drug, for example, means a nontoxic but sufficient amount of a drug to provide the selected effect over a specific period of time or number of doses.

It will be appreciated that an amount that constitutes a effective amount will vary according to the particular active incorporated in the tablet, the condition and/or severity of the condition being treated, any other actives being co-administered with the selected active, other components of the tablet, desired duration of treatment and preferred number of dosage units, age of the individual to which the tablet is being administered, the size of the tablet, and the like.

A single dosage form will typically be a single tablet but may refer to multiple tablets administered at substantially the same time. In this regard it is common in the medicinal art that one or two tablets are recommended, e.g., depending on body weight, age, or the like, for administration as a single dose.

The tablets of the invention are used to administer a desired predetermined substance, referred to herein as an "active", an "active ingredient", an "active agent", and the like, at levels sufficient or effective to impart a desired action or specific intended dose. The term active, also referred to herein as an active ingredient, is used to mean any "drug", "bioactive agent," "preparation," "medicament," "therapeutic agent," "physiological agent," "nutraceutical," "flavor," or "pharmaceutical agent" and include substances for use in the diagnosis, cure, mitigation, arrest, treatment or prevention of a condition or disease state or to affect the structure or function of the body. Dietary supplements, functional foods (e.g., ginger, green tea, lutein, garlic, lycopene, capsaicin, and the like)

Tablets comprising the bioadhesive composition and a drug or other active with drug loads of from about 0.01 % to about 80%, more typically from about 0.1 % to about 70%, even more typically from about 1 to about 60 %, may be manufactured and used to deliver active agent to an individual to which the tablet is administered. It will be appreciated that drug loading will depend on the drug and the bioadhesive used. The loading of, e.g., benzocaine, will typically be about 7.5 %.

The term "bioadhesive system" as used herein includes any system or product, including the tablet described herein, that comprises a bioadhesive composition and active agent(s) to be delivered.

Bioadhesive compositions used to prepare the tablets of the invention include mixtures of starches, preferably pregelatinized or physically, chemically or enzymatically modified starches, and synthetic polymer or other excipient necessary for optimization of the formulation such as, for example, polyacrylic acid, hydroxyethylcellulose, polyvinyl alcohol, sodium carboxymethylcellulose, polyvinylpyrrolidone and polyethylene glycol.

The starch may be pregelatinized for immediate use in preparing the bioadhesive or, alternatively, may be pregelatinized and then stored for later use in preparing the bioadhesive. The starch may be jet cooked or batch cooked.

Methods of preparing bioadhesive compositions used to prepare the tablets of the invention are known in the art. E.g., a bioadhesive composition may be accomplished by preparing a solution by charging at least one solvent, preferably water, and a polymer mixture such as a mixture of a polysaccharide and a natural and/or synthetic polycarboxylated polymer into a reaction vessel. In order to partially or totally solubilize the mixture, the solution may be heated and stirred for a short period. The mixture can then be dried by conventional means, including, but not limited to, spray drying, freeze drying, air drying, drum drying and extrusion, to provide a solid. The polycarboxylated polymer may be treated with cations to effect changes in pH and/or viscosity, as would be apparent to one skilled in the art. The concentrations of the component polymer solutions are determined only by consideration of degree of solubility and a convenient viscosity for mixing and subsequent processing, as will be obvious to one skilled in the art.

A preferred bioadhesive composition for use in preparing the tablets of the invention is one prepared by drying of a polycarboxylated polymer and a polysaccharide under conditions that result in an intimate mixture thereof, as opposed to a mere physical mixture.

Intimate mixture is used herein to refer to mixtures wherein each particle comprises a mixture of, e.g., starch and poly(acrylic acid). This is in contrast to a physical mixture, which refers to mixtures comprising discrete particles of e.g., starch and poly(acrylic acid).

Co-spray drying polymer mixtures has been found useful in preparing such intimate mixtures. Such a procedure is described in U.S. Patent Application Publication No. US 2003/0143277.

Preferably, the ratio of polymers in the solution mixture lies within the range of about 5 parts (on a dry weight basis) polysaccharide plus 95 parts polycarboxylated polymer to about 95 parts polysaccharide plus 5 parts polycarboxylated polymer. Preferably, the ratio lies within the range of about 25 parts polysaccharide plus 75 parts polycarboxylated polymer to about 95 parts polysaccharide plus 5 parts polycarboxylated polymer. More preferably, for application to the mucosal surface, the ratio lies within the range of about 65 parts polysaccharide plus 35 parts polycarboxylated polymer to about 95 parts polysaccharide plus 5 parts polycarboxylated polymer. Even more preferably, the ratio will be from about 75 parts polysaccharide plus 25 parts polycarboxylated polymer to about 95 parts polysaccharide plus 5 parts polycarboxylated polymer. It has, however, also been found that partial neutralization of the comixture prevents denaturing of peptides and proteins such as insulin and permits use of high levels (e.g., 75%) of polycarboxylated polymer without inducing irritation.

The mixture is then dried by conventional means, including, but not limited to, spray drying, freeze drying, air drying, drum drying and extrusion, to provide a solid (e.g., a powder). The solid produced during the drying stage will preferably have a moisture content of less than about 13 % by weight, preferably less than about 9 % by weight. A particularly preferred method is spray drying.

The conditions used to prepare the bioadhesive compositions of the invention are sufficiently mild and/or the processing sufficiently rapid that unwanted chemical reactions, that may lead to deleterious byproducts, are avoided. Thus, no purification step is needed to remove such components.

The bioadhesion compositions, e.g., prior to incorporation of an active ingredient, may be neutralized by known means, if desired.

Synthetic polycarboxylated polymers particularly useful in the practice of this invention may be modified or unmodified and have a weight average molecular weight of at least 10,000 Daltons, more typically at least about 100,000 Daltons, even more typically above about 1,000,000 Daltons. Modifications may include, but are not limited to cross-linking, neutralization, hydrolysis and partial esterification.

Exemplary synthetic polycarboxylated polymers which may be used in the present invention include without limitation poly(acrylic acid), cross-linked poly(acrylic acid), poly(acrylic acid) modified by long chain alkyl acrylates, cross-linked poly(acrylic acid) modified by long chain alkyl acrylates. Typical synthetic polycarboxylated polymers of this invention include acrylic acid polymers crosslinked with allyl sucrose, allyl ethers of sucrose, allylpentaerythritol, pentaerythritol or divinyl glycol. Such polymers are available from NOVEON under the trade names CARBOPOL^{®}, NOVEON^{®} and PEMULEN^{®}. Particularly suitable are the pharmaceutical grades CARBOPOL^{®} 971 P, CARBOPOL^{®} 934P and CARBOPOL^{®} 974P. These examples are not limiting and the polysaccharides according to the present invention may be used in combination with virtually any synthetic polycarboxylated polymer.

Useful polysaccharides may be derived from natural products, including plant, animal and microbial sources. Examples of polysaccharides include starch, cellulose and gums such as galactomannans. Polysaccharide starches include maize or corn, waxy maize, potato, cassava, tapioca and wheat starch. Other starches include varieties of rice, waxy rice, pea, sago, oat, barley, rye, amaranth, sweet potato, and hybrid starches available from conventional plant breeding, e.g., hybrid high amylose starches having amylose content of 40 % or more, such as high amylose corn starch. Also useful are genetically engineered starches such as high amylose potato and waxy potato starches. The polysaccharides may be modified or derivatized, such as by etherification, esterification, acid hydrolysis, dextrinization, crosslinking, pregelatinization or enzyme treatment (e.g., with *alpha*-amylase, *beta-*amylase, pullulanase, isoamylase, or glucoamylase). Particularly preferred are waxy starches. As used herein, the term "waxy" is intended to include a starch containing at least about 95 % by weight amylopectin.

Preferred polysaccharides will have a weight average molecular weight of at least 10,000 Daltons, more preferably at least about 100,000 Daltons, even more preferably above about 500,000 Daltons, and most preferably greater than about 1,000,000 Daltons. While molecular weights of waxy starches are difficult to determine, waxy starches that can be used in the practice of the invention may have weight average molecular weights of 10,000,000 Daltons or more.

The tablet of the invention may also comprise other optional ingredients such as plasticizers, emulsifiers, humectants, surfactants, colorants, proteins, flavors, flavor enhancers sweeteners, and masking agent. It will be appreciated that flavors and/or sweeteners in one embodiment may be an active, while a flavor and/or sweetener in other embodiment may be used, e.g., as a masking agent.

Other optional components may be added for a variety of reasons including, without limitation, sweeteners, both natural and artificial; emulsifiers such as Polysorbate 80; humectants; surfactants; masking agent such as flavorings; colorants, more particularly food grade colors; proteins such as gelatins; and in addition to flavors, flavor enhancers. Another optional component of the tablet is a gelling agent. Gelling agents include, but are not limited to carageenan, gellan gums, locust bean, tragacanth gum, guar gum, acacia gum, and arabic gum. These optional components are typically added in minor amounts, particularly less than about 30% total by weight based upon the weight of the final formulated product.

The tablet of the invention is well suited for application to the mucosa. The tablet may be adhered to the oral cavity, or other mucosal surface where it releases a pharmaceutically or cosmetically active agent. The tablet is well suited for the delivery of a wide range of pharmaceutically active ingredients via the mucous membranes of a patient, particularly the buccal mucosa. Therapeutic agents that exhibit absorption problems due to solubility limitations, degradation in the gasto-intestinal tract, or extensive metabolism are particularly well suited.

The bioadhesive may be used to deliver a therapeutically effective amount of a product to the mucosa of a patient, to deliver a therapeutically effective amount of drug across the mucosa of a patient, or to deliver therapeutically effective amount of a product to the vicinity of the tablet (e.g., the oral cavity). The tablet can be applied to normal mucosa, or to damaged or irritated or diseased mucosal tissue.

The term bioadhesive administration refers to the use of the mucosa as a portal for the administration of drugs by topical application or for diagnostic procedures such as the monitoring of blood chemistry. The topically applied drug passes into and/or through the mucosa. This term is used broadly to refer to the topical administration of a drug which acts locally, i.e., at the surface or within the mucosa, such as, for example, a patch used to treat a sore in the mouth, and to the topical application of a drug which acts systemically by diffusing through the skin and entering the blood stream.

It will be appreciated that components of the bioadhesive tablet can be selected to control the time and degree or extent of erosion over time. Particularly preferred bioadhesive tablets are completely bioerodable.

The shape of tablet of the invention is defined by the ratio of the tablet diameter *D* to the cup depth *d* (*D*/*d*) and by the ratio of cup depth *d* to edge thickness *h* (*d*/*h*). Figures 1 and 2 are enlarged drawings of tablets having a diameter (*D*) of 0.4375 inch, a depth (*d*) of 0.040 inch and an edge thickness (h) of 0.01 inch. In the tablet of Figure 1, the edge thickness is calculated as *h=t-2d.* In the tablet of Figure 2, the edge thickness is calculated as *h=t-d.*

Tablets for use in the practice of the invention require a *D*/*d* in the range 4-20, more preferably 4-14, and require a *d*/*h* greater than 0.75, more preferably greater than 0.90. In one embodiment the tablet has a *d*/*h* greater than about 1.00. Tablets with this shape show a marked improvement in reliability and durability of adhesion compared with flat profile tablets and are more comfortable in the mouth.

Tablets having non-circular shape, for example an elliptical, approximately elliptical or oval shape or a rectangular shape with rounded corners are also encompassed by the invention. For the purposes of this invention the tablet diameter D is defined to be the geometric mean of the diameter measured along the major and minor axes of the ellipse or similar shape. For example, an elliptical tablet having a semi-major axis equal to 6 mm and a semi-minor axis equal to 4 mm will have D = √(12 mm x 8 mm) = 9.80 mm.

Figure 1 shows a symmetric, convex both sides, tablet. However, it is not necessary that both sides have the same depth d, provided that at least one side satisfies the condition that *d*/*h* is greater than 0.75.

It will be appreciated that the size and weight of the tablet will vary depending of the site of delivery and the individual (e.g., human, dog, horse) being treated. Tablets will generally range from 20 to about 2,000 mg. Tablets for buccal delivery will generally range from about 100 to about 200 mg in weight, more typically from about 130 to about 200 mg, with a bioadhesive component containing Amioca and Carbopol. The Carbopol content is preferably between 10 and 30 wt %, more preferably between 15 and 25 wt %. A particularly preferred bioadhesive component will contain Amioca and Carbopol in a 75:25 ratio by weight. Known processing aids may be added as necessary, for example a flow aid such as fumed silica or a lubricant such as magnesium stearate.

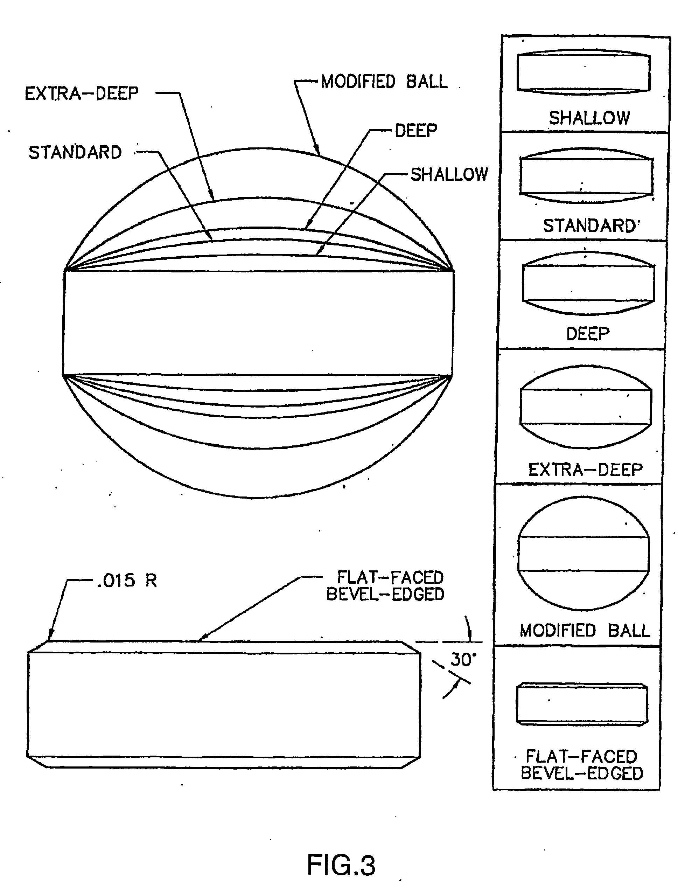

Conventionally shaped tablets have a profile whose geometry is defined in the Tableting Specification Manual (TSM) of the American Pharmaceutical Association. The profiles of the following conventional tablets: shallow cup, standard cup, deep cup, extra deep cup, modified ball, and flat-faced bevel-edged is shown in drawing Figure 3 (adapted from TSM of the American Pharmaceutical Association, 6^{th} edition, 2003, page 52).

Punch tip dimensions and calculation of punch diameter D / depth d ratios for punches of the varying curvatures of drawing Figure 1 are shown in Tables 1-5.

**Table 1**

| (Shallow Cup) | | |
|---|---|---|
| Diameter D (mm) | Depth d (mm) | D/d |
| 3.175 | 0.127 | 25.000 |
| 3.970 | 0.178 | 22.303 |
| 4.763 | 0.203 | 23.463 |
| 5.555 | 0.229 | 24.258 |
| 6.350 | 0.254 | 25.000 |
| 7.142 | 0.305 | 23.416 |
| 7.938 | 0.330 | 24.055 |
| 8.730 | 0.356 | 24.522 |
| 9.525 | 0.406 | 23.461 |
| 10.318 | 0.432 | 23.884 |
| 11.113 | 0.457 | 24.317 |
| 11.905 | 0.508 | 23.435 |
| 12.700 | 0.533 | 23.827 |
| 13.493 | 0.559 | 24.138 |
| 14.288 | 0.610 | 23.423 |
| 15.080 | 0.635 | 23.748 |
| 15.875 | 0.660 | 24.053 |
| 17.463 | 0.737 | 23.695 |
| 19.050 | 0.787 | 24.206 |
| 20.638 | 0.864 | 23.887 |
| 22.225 | 0.940 | 23.644 |
| 23.813 | 0.991 | 24.029 |
| 25.400 | 1.067 | 23.805 |

**Table 2**

| (Standard Cup) | | |
|---|---|---|
| Diameter D (mm) | Depth d (mm) | D/d |
| 3.175 | 0.432 | 7.350 |
| 3.970 | 0.533 | 7.448 |
| 4.763 | 0.635 | 7.501 |
| 5.555 | 0.737 | 7.537 |
| 6.350 | 0.787 | 8.069 |
| 7.142 | 0.838 | 8.523 |
| 7.938 | 0.864 | 9.188 |
| 8.730 | 0.889 | 9.820 |
| 9.525 | 0.914 | 10.421 |
| 10.318 | 0.965 | 10.692 |
| 11.113 | 1.016 | 10.938 |
| 11.905 | 1.041 | 11.436 |
| 12.700 | 1.092 | 11.630 |
| 13.493 | 1.143 | 11.805 |
| 14.288 | 1.168 | 12.233 |
| 15.080 | 1.219 | 12.371 |
| 15.875 | 1.270 | 12.500 |
| 17.463 | 1.372 | 12.728 |
| 19.050 | 1.473 | 12.933 |
| 20.638 | 1.549 | 13.323 |
| 22.225 | 1.651 | 13.462 |
| 23.813 | 1.753 | 13.584 |
| 25.400 | 1.854 | 13.700 |

**Table 3**

| (Deep Cup) | | |
|---|---|---|
| Diameter D (mm) | Depth d (mm) | D/d |
| 3.175 | 0.610 | 5.205 |
| 3.970 | 0.762 | 5.210 |
| 4.763 | 0.914 | 5.211 |
| 5.555 | 1.067 | 5.206 |
| 6.350 | 1.143 | 5.556 |
| 7.142 | 1.168 | 6.115 |
| 7.938 | 1.194 | 6.648 |
| 8.730 | 1.245 | 7.012 |
| 9.525 | 1.270 | 7.500 |
| 10.318 | 1.321 | 7.811 |
| 11.113 | 1.372 | 8.100 |
| 11.905 | 1.422 | 8.372 |
| 12.700 | 1.499 | 8.472 |
| 13.493 | 1.549 | 8.711 |
| 14.288 | 1.600 | 8.930 |
| 15.080 | 1.676 | 8.998 |
| 15.875 | 1.727 | 9.192 |
| 17.463 | 1.854 | 9.419 |
| 19.050 | 1.981 | 9.616 |
| 20.638 | 2.108 | 9.790 |
| 22.225 | 2.260 | 9.830 |
| 23.813 | 2.388 | 9.972 |
| 25.400 | 2.515 | 10.099 |

**Table 4**

| Extra Deep Cup | | |
|---|---|---|
| Diameter D (mm) | Depth d (mm) | D/d |
| 3.175 | 0.762 | 4.162 |
| 3.970 | 0.914 | 4.344 |
| 4.763 | 1.067 | 4.464 |
| 5.555 | 1.219 | 4.557 |
| 6.350 | 1.270 | 5.000 |
| 7.142 | 1.372 | 5.026 |
| 7.938 | 1.524 | 5.209 |
| 8.730 | 1.676 | 5.209 |
| 9.525 | 1.829 | 5.208 |
| 10.318 | 1.981 | 5.208 |
| 11.113 | 2.134 | 5.208 |
| 11.905 | 2.286 | 5.208 |
| 12.700 | 2.413 | 5.263 |
| 13.493 | 2.565 | 5.260 |
| 14.288 | 2.718 | 5.257 |
| 15.080 | 2.870 | 5.254 |
| 15.875 | 3.023 | 5.251 |
| 17.463 | 3.327 | 5.249 |
| 19.050 | 3.632 | 5.245 |
| 20.638 | 3.937 | 5.242 |
| 22.225 | 4.220 | 5.267 |
| 23.813 | 4.547 | 5.237 |
| 25.400 | 4.851 | 5.236 |

**Table 5**

| Modified Ball | | |
|---|---|---|
| Diameter D (mm) | Depth d (mm) | D/d |
| 3.175 | 1.016 | 3.125 |
| 3.970 | 1.245 | 3.189 |
| 4.763 | 1.499 | 3.177 |
| 5.555 | 1.753 | 3.169 |
| 6.350 | 2.007 | 3.164 |
| 7.142 | 2.261 | 3.159 |
| 7.938 | 2.515 | 3.156 |
| 8.730 | 2.769 | 3.153 |
| 9.525 | 3.023 | 3.151 |
| 10.318 | 3.251 | 3.174 |
| 11.113 | 3.378 | 3.290 |
| 11.905 | 3.759 | 3.167 |
| 12.700 | 4.013 | 3.165 |
| 13.493 | 4.267 | 3.162 |
| 14.288 | 4.521 | 3.160 |
| 15.080 | 4.775 | 3.158 |
| 15.875 | 5.029 | 3.157 |
| 17.463 | 5.512 | 3.168 |
| 19.050 | 6.020 | 3.164 |
| 20.638 | 6.528 | 3.161 |
| 22.225 | 7.036 | 3.159 |
| 23.813 | 7.518 | 3.167 |
| 25.400 | 8.026 | 3.165 |

Conventional tablets, as determined from drawing Figure 1, have the ratio *d*/*h* shown in Table 6.

**Table 6**

| Cup type | *d*/*h* |
|---|---|
| Shallow | 0.17 |
| Standard | 0.32 |
| Deep | 0.44 |
| Extra deep | 0.72 |
| Modified ball | 1.22 |

It can be seen from this data that none of the conventional shallow cup, standard cup, deep cup, extra deep cup, modified ball or flat-faced bevel-edged tablets meet the requirements for use in the practice of the invention. Since tablets suitable for use in the practice of the invention must have a ratio *D*/*d* in the range 4-20, shallow cup and modified ball tablet designs are excluded for use in the invention. Since tablets suitable for use in the practice of the invention must also have a *d*/*h* greater than 0.75, shallow cup, standard cup, deep cup, and extra deep cup designs are excluded for use in the practice of the invention.

The invention will be described further in the following examples, which are included for purposes of illustration and are not intended, in any way, to be limiting of the scope of the invention.

### EXAMPLES

### EXAMPLE 1

This example describes the preparation of a bioadhesive composition prepared by co-spray drying a mixture of Amioca and CARPOPOL^{®}.

A mixture of 10% by weight of Amioca waxy corn starch (obtained from National Starch & Chemical Company, Bridgewater, New Jersey) and 90% water was prepared as a slurry. The mixture was heated by injecting steam at a pressure of 2.75 bar in a continuous jet cooker, maintaining the temperature at 150 °C by adjustment with jacketed cooling water. The final starch solids content, determined by heating a small sample for 2 hours at 135 °C, was 7.74%.

A 1 % aqueous solution of CARBOPOL^{®}974P (obtained from Noveon, Inc.) was prepared by slowly adding the CARBOPOL to deionized water while continuously stirring until completely dispersed.

The starch and CARBOPOL solutions were uniformly mixed in such proportions as to obtain the desired ratio of starch to CARBOPOL. For example, mixing 1085 g Amioca solution with 5600 g CARBOPOL solution yielded a ratio of 60% Amioca to 40% CARBOPOL, calculated on solids basis. The solution mixture was heated in a water bath to 40 °C and spray dried using a centrifugal wheel atomizer. The inlet temperature during drying was 205 °C and the outlet temperature 110 °C. The resulting product was a fine, low density, white powder comprising an intimate mixture of Amioca and CARBOPOL.

### Example 2

Using a spray-dried mixture of 85% Amioca to 15% CARBOPOL, a series of convex (both sides) shaped tablets where made with reduced mass to decrease the edge thickness. A Standard punch, D = 0.4375" and *d* = 0.040" was used. Calculated edge thickness, *h,* and the ratios of *d*/*h* are shown in Table 7.

**Table 7**

| Mass (mg) Mass(mg) | Measured thickness in center (in) *t* | Calculated edge thickness (in) *h* | *d*/*h* |
|---|---|---|---|
| 196 | 0.122 | 0.042* | 0.95 |
| 180 | 0.116 | 0.036 | 1.11 |
| 160 | 0.1125 | 0.0325 | 1.23 |
| 140 | 0.106 | 0.026 | 1.54 |
| 120 | 0.102 | 0.022 | 1.82 |
| 130 | 0.1045 | 0.0245 | 1.63 |

| | | | |
|---|---|---|---|
| *Calculated from measured thickness in center and d of punch used to be: h=t-2d or 0.122-(2x0.040)=0.042. | | | |

### Example 3

A bioadhesive tablet, having a mass of 130 mg and containing 15 mg micronized benzocaine, was prepared. The bioadhesive component was a co-spray dried mixture of jet cooked Amioca and Carbopol 974P NF. The ingredients that were used in preparing the tablets are shown in Table 8.

**Table 8**

| Component | Mass (mg) | Mass (wt.%) |
|---|---|---|
| Amioca | 86.25 | 66.35 |
| Carbopol | 28.75 | 22.11 |
| Benzocaine | 15.00 | 11.54 |
| Total | 130.00 | 100.00 |

A tablet size of 0.4375" diameter was prepared by compression at 3000 psi with a pair of standard cup punches (cup depth 0.040"). This produced a tablet with a thickness about 0.1045" measured in the center and about 0.0245" at the edge. The ratio of cup to edge depth was 1.6.

## Claims

1. A tablet having at least one convex surface, said tablet having a ratio of tablet diameter D to the cup depth *d* (*D*/*d*) of 4-20 and a ratio of cup depth d to edge thickness *h* (*d*/*h*) of greater than 0.75.

2. The tablet of claim 1 wherein the ratio of tablet diameter D to the cup depth *d* (*D*/*d*) is from 4-14 and ratio of cup depth d to edge thickness *h* (*d*/*h*) of greater than 0.90.

3. The tablet of claim 1 comprising a bioadhesive composition and an active.

4. The tablet of claim 3 wherein the bioadhesive composition comprises a mixture of a polysaccharide and a polycarboxylated polymer.

5. The tablet of claim 4 wherein said mixture is a co-spray-dried mixture.

6. The tablet of claim 4 wherein the bioadhesive composition comprises from about 15 % by weight to about 25 % by weight of at least one synthetic polycarboxylated polymer.

7. The tablet of claim 6 wherein the polysaccharide is a starch and the polycarboxylated polymer is a cross-linked poly(acrylic acid).

8. The tablet of claim 6 wherein the starch is a waxy starch.

9. A method of producing a bioadhesive system comprising preparing a solution comprising at least one solvent and a polymer mixture wherein the polymer mixture comprises at least one synthetic polycarboxylated polymer component and at least one polysaccharide component and drying the solution to form a solid, and compressing the solid to form a tablet, said tablet having at least one convex surface and having a ratio of tablet diameter *D* to the cup depth *d* (*D*/*d*) of 4-20 and a ratio of cup depth *d* to edge thickness *h* (*d*/*h*) of greater than 0.75.

10. The method of claim 9 wherein the solution is dried by spray-drying.

11. The method of claim 10 wherein the solvent is water, the polysaccharide is a starch and the polycarboxylated polymer is cross-linked poly(acrylic acid).

12. A bioadhesive system prepared by the method of claim 9.

13. The bioadhesive system of claim 12 further comprising a drug as the active ingredient.

14. A method of administering a therapeutic agent to an individual comprising administering to the individual the tablet of claim 1, which tablet contains a therapeutic agent.

15. The method of claim 14 wherein the tablet is applied to a mucosal surface of the individual.

16. The method of claim 15 wherein the tablet is administered to the buccal, intestinal, nasal, ocular, sub-lingual, vaginal or rectal mucosa.

17. A method of administering an active ingredient to an individual needing or desiring said active ingredient comprising applying the active-containing tablet of claim 3 to a mucosal surface of said individual.

18. The method of claim 17 wherein the therapeutic agent is benzocaine.

19. A buccal delivery tablet comprising a bioadhesive composition and an active agent, tablet having at least one convex surface, said tablet having a ratio of tablet diameter *D* to the cup depth *d* (*D*/*d*) of 4-20 and a ratio of cup depth *d* to edge thickness *h* (*d*/*h*) of greater than 0.75.

20. The tablet of claim 19 wherein the active agent is benzocaine.
